# EUROPEAN PATENT SPECIFICATION

(11) **EP 3 609 554 B1**
(45) Date of publication and mention of the grant of the patent: **09.06.2021**
(21) Application number: 18718101.1
(22) Date of filing: 10.04.2018
(51) Int. Cl.: A61M 3/02

(54) **IRRIGATION SYSTEM WITH PRESSURE SENSING AND CONTROL**
BEWÄSSERUNGSSYSTEM MIT DRUCKMESSUNG UND -STEUERUNG
SYSTÈME D'IRRIGATION AYANT UNE DÉTECTION ET UNE COMMANDE DE PRESSION

(30) Priority: 10.04.2017 DK PA201770260
(43) Date of publication of application: 19.02.2020
(73) Proprietor: Coloplast A/S, 3050 Humlebaek (DK)
(72) Inventor: HVID, Niels, 2950 Vedbaek (DK); BAY, Henrik, 2800 Lyngby (DK)
(86) International application number: PCT/DK2018/050073
(87) International publication number: WO 2018/188710

(56) References cited:
- EP-A1- 1 547 630
- EP-A1- 1 946 785
- EP-A2- 0 513 858
- WO-A1-2015/029039

## Description

The present disclosure relates to an irrigation system for anal and/or stomal irrigation and related method. In particular, a method of operating an irrigation system for anal and/or stomal irrigation is disclosed.

### Background

Control of voluntary bowel functions is frequently limited or absent in patients suffering from certain disabilities, such as spinal injuries, multiple sclerosis or spina bifida. Such lack of control of voluntary bowel functions typically results in faecal incontinence or intractable constipation, as patients have significantly reduced ability to sense the presence of faeces in the colon terminal part and the rectum and to sense the evacuation stimulus. Patients having undergone stomal surgery wherein a catheterizable stoma is constructed may suffer from similar difficulties.

It is known to accomplish bowel emptying by irrigation (i.e. flushing) of the rectum or stoma, by an irrigating fluid, such as tap water or saline, which is provided through an intermittent catheter with a tip which is configured and sized for insertion into the rectum or stoma, where it remains in a fixed position by an expandable inflation element, such as a balloon. The balloon may be inflatable by air or by water. Once the rectum or stoma has been flushed with the irrigation liquid, the expandable retention element is allowed to collapse to its non-deflated state, allowing the catheter to be withdrawn from the rectum or stoma, and allowing irrigation liquid and faeces to evacuate. The catheter is connected to a reservoir of irrigation liquid through a tube, and a pump may be provided for displacing or pumping irrigation liquid from the reservoir to the catheter.

WO2015/029039 discloses systems and methods for cleaning a colon or other portion of an intestine include use of sensors to detect conditions of blockage of flow of materials within an evacuation channel used to remove fecal material from the body, and devices and methods for purging such blockages from the evacuation channel.

EP1946785 discloses an irrigation system comprising a reservoir and an insertion member and a liquid tube fluidly connecting the reservoir and the insertion member.

EP0513858 disclose a tubing set for surgical irrigation systems for treatment of wound or joint for relief of pain and promotion of healing.

EP1547630 discloses a double membrane transducer protector.

### Summary

There is a need for systems and//or methods increasing the safety for a user of an irrigation system and/or reduce the risk of damage or irritation of a user's bowel during and/or after irrigation. The invention is defined by the features of independent claim 1. The disclosed methods do not fall under the scope of the claimed subject matter.

Accordingly, an irrigation system for anal and/or stomal irrigation is provided, the irrigation system comprising a reservoir for an irrigation liquid; a tubing system with a first end connectable to the reservoir, and a second end connectable to a catheter for provision of a first fluid path for the irrigation liquid between the reservoir and the catheter comprising a distal end for expelling irrigation liquid from the catheter; one or more pressure sensors including a first pressure sensor for provision of a first pressure signal; a pump operable to pump the irrigation liquid from the reservoir to the second end of the tubing system; and a control system connected to the first pressure sensor and configured to operate the pump based on the first pressure signal. The tubing system optionally comprises a pressure channel and a first membrane. The first membrane can be arranged between the first fluid path and the pressure channel, and optionally the first pressure sensor is configured to measure the pressure in the pressure channel.

Also, a method of operating an irrigation system for anal and/or stomal irrigation is provided. The irrigation system optionally comprises a reservoir for an irrigation liquid; a tubing system with a first end connectable to the reservoir, and a second end connectable to a catheter for provision of a first fluid path for the irrigation liquid between the reservoir and the catheter comprising a distal end for expelling irrigation liquid from the catheter; one or more pressure sensors including a first pressure sensor for provision of a first pressure signal; a pump operable to pump the irrigation liquid from the reservoir to the second end of the tubing system; and a control system connected to the first pressure sensor and configured to operate the pump based on the first pressure signal, the tubing system comprising a pressure channel and a first membrane, wherein the first membrane is arranged between the first fluid path and the pressure channel, and the first pressure sensor is configured to measure the pressure in the pressure channel. The method comprises: pumping irrigation liquid from the reservoir through the first fluid path; measuring the pressure in the pressure channel, and reducing the irrigation liquid pressure in the first fluid path if the pressure in the pressure channel is larger than a first pressure threshold.

The present disclosure enables more safe anal and/or stomal irrigation by improving the pressure control of the irrigation liquid flow in the irrigation system. The present disclosure enables more effective irrigation while at the same time providing a safe irrigation, by improving the accuracy of fluid pressure control in the irrigation system. Current systems, in order to comply with safety regulations, typically operate with a large safety margin, which in turn reduces the irrigation efficiency in certain use situations. The safety margins applied may result in irrigation liquid pressure at the catheter tip below a desired minimum pressure, e.g. in the range from 1.2 to 1.6 psi, for certain use situations. It is an advantage of the present disclosure that the liquid pressure control is improved, e.g. by increasing the minimum liquid pressure of the irrigation liquid at the catheter tip.

The present disclosure provides a simple, efficient, and easy-to-use irrigation system with a high degree of safety for a user, in particular for a POC system that can be used in many setups/situations.

### Brief Description of the Drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Fig. 1 illustrates an exemplary irrigation system,
Fig. 2 illustrates an exemplary irrigation system,
Fig. 3 illustrates a part of an exemplary tubing system with a catheter,
Fig. 4 schematically illustrates a part of an exemplary irrigation system,
Fig. 5 schematically illustrates a part of an exemplary irrigation system, and
Fig. 6 shows a more detailed view of a part of the second tubing part.

### Detailed Description

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

An irrigation system for anal and/or stomal irrigation is disclosed. The irrigation system may be a point-of-care (POC) irrigation system. A POC irrigation system must be safe to operate with low or no risk of errors, in particular with no risk of self-damaging of a user. The irrigation system may be an irrigation system with no return flow of fluid and faecal material in the tubing system of the irrigation system.

The irrigation system comprises a reservoir for an irrigation liquid. The reservoir may be arranged in a reservoir housing. The reservoir may be configured to hold irrigation liquid having a volume of in the range from 100 mL to 3.0 L. In one or more exemplary irrigation systems, the reservoir has a reservoir volume less than 3 L.

The irrigation system optionally comprises a user interface, optionally arranged in a user interface housing and/or the reservoir housing. The user interface is configured to control operation of the irrigation system, e.g. by sending "start", "stop", and/or "pause" control signals to the control system in response to a user providing user input, e.g. by pressing one or more buttons of the user interface. The user interface may comprise a touch sensitive surface, such as a touch-screen for receiving user input.

The irrigation system comprises a tubing system with a first end and a second end. The first end is optionally connectable or connected to the reservoir and the second end is optionally connectable or connected to a catheter or an adapter for provision of a first fluid path for the irrigation liquid between the reservoir and the catheter comprising a distal end for expelling irrigation liquid from the catheter. The irrigation system may comprise a first fluid path from the first end of the tubing system to the second end of the tubing system.

The irrigation system may comprise a second fluid path. The second fluid path may extend to the second end of the tubing system. The second fluid path may extend from the first end of the tubing system to the second end of the tubing system. The second fluid path may be parallel to the first fluid path for feeding second fluid to the second end of the tubing system.

The tubing system may comprise one or more tubing parts, such as a first tubing part and/or a second tubing part, for forming fluid path(s) between different housings of the irrigation system. The tubing system and parts thereof may comprise one or more first channels for forming parts of the first fluid path. The tubing system and parts thereof may comprise one or more second channels for forming parts of a second fluid path. The first fluid path of the irrigation system is for feeding irrigation liquid from the reservoir to the second end of the tubing system, e.g. for irrigating the bowel of the user. The second fluid path of the irrigation system may be for feeding irrigation liquid from the reservoir to the second end of the tubing system. The second fluid path may be for inflating or filling a balloon of the catheter, e.g. with irrigation liquid from the reservoir and/or air.

The tubing system may comprise a first connector at the first end of the tubing system. The tubing system may comprise a second connector at the second end of the tubing system. A tubing system with one or more connectors facilitate the use of different sized tubing system, e.g. to adapt to different users, and/or easy handling of the irrigation system, e.g. disassembly for storing. The second connector may be configured for coupling, e.g. detachable coupling, to a catheter and/or a catheter adapter.

The irrigation system comprises one or more pressure sensors including a first pressure sensor for provision of a first pressure signal. The one or more pressure sensor may comprise a plurality of pressure sensors. The one or more pressure sensors may include a second pressure sensor for provision of a second pressure signal. The control system may be connected to the second pressure sensor. The control system may be configured to operate the pump based on the second pressure signal. A second pressure sensor increases the robustness of the pressure control and/or reduces the risk of errors in the irrigation system. The second pressure sensor may be arranged in a user interface housing or in the reservoir housing. The one or more pressure sensors may include a third pressure sensor for provision of a third pressure signal. The control system may be connected to the third pressure sensor. The control system may be configured to operate the pump based on the third pressure signal. The third pressure sensor may be arranged in the user interface housing or in the reservoir housing.

The first pressure sensor provides a first pressure signal. The first pressure signal may be indicative of relative pressure between the pressure in the pressure channel and an ambient pressure. The first pressure signal may be indicative of absolute pressure in the pressure channel. The first pressure sensor may be arranged in the user interface housing or in the reservoir housing.

The irrigation system comprises a pump, such as an electrical pump, operable to pump the irrigation liquid from the reservoir to the second end of the tubing system. The pump may be arranged with the reservoir in the reservoir housing. Arranging the pump in the reservoir housing allows for a small user interface housing. Arranging the pump in the reservoir housing allows for a more precise control of the irrigation flow in the first fluid path. The pump may be arranged in the user interface housing. Arranging the pump in the user interface housing allows for a more simple first tubing part with no wired connection.

The irrigation system comprises a control system comprising a controller connected to the first pressure sensor. The control system may be configured to operate the pump based on the first pressure signal. The control system may be configured to operate the pump based on the second pressure signal. The control system may be configured to operate the pump to reduce the irrigation liquid pressure in the first fluid path, if the pressure in the pressure channel is larger than a first pressure threshold and/or fulfils a pressure criterion.

The tubing system comprises a pressure channel and a first membrane, wherein the first membrane is arranged between the first fluid path and the pressure channel. The first membrane is flexible such that the liquid pressure in the first fluid path is transferred, e.g. at least within a first pressure range, to the pressure channel. The tubing system may comprise a second connector at the second end, and the first membrane may be arranged in the second connector. The first membrane may also be denoted a diaphragm and operates to eliminate, minimize or reduce the pressure difference between the pressure in the first fluid path and the pressure in the pressure channel. Thereby, the liquid pressure in the first fluid path at the position of the first membrane can be translated to a pressure sensor at a distance from the first membrane via the pressure channel. The pressure channel may be filled with air, thereby avoiding the pressure contribution from a liquid in the pressure channel.

The second connector comprises one or more connector parts including a first connector part, optionally a second connector part, optionally a third connector part 46. The first connector part may, in an assembled second connector, be optionally snap-locked to and/or in threaded engagement with the second connector part. The second connector part may, in an assembled second connector, be optionally snap-locked to and/or in threaded engagement with the third connector part. The second connector has a first outlet of first fluid path and optionally a second outlet of the second fluid path (if present) of the tubing system 6. The first membrane may be arranged in an expansion chamber of the first connector part. Thereby, the surface area of the flexible first membrane is sufficiently large to reduce, minimize or eliminate the pressure difference between the first fluid path and the pressure channel. The first membrane separates the first fluid channel and the pressure channel. The first membrane is flexible to substantially equalize the pressure in the first fluid path and the pressure in the pressure channel, e.g. at least within a pressure difference less than a pressure difference threshold. The pressure difference threshold may be in the range from 0 to 0.5 psi, such as less than 0.2 psi. The second connector may have a first channel forming a part of the first fluid path, the first channel ending in the first outlet. The second connector may have a second channel forming a part of the second fluid path, the second channel ending in the second outlet.

The second connector may comprise a first window between a first channel in the second connector and the pressure channel, the first channel in the second connector optionally forming part of the first fluid path. The first window may be sealed by the first membrane. The first membrane may be arranged at a distance less than 50 cm from the second end of the tubing system. The first membrane may have a first surface with a first surface area, the first surface facing the first fluid path. The first membrane may have a second surface with a second surface area, the second surface facing the pressure channel. The first membrane may have a first surface area less than 5 cm², such as in the range from 10 mm² to 4 cm². The first membrane may have a thickness less than 1 mm.

The first pressure sensor is optionally configured to measure the pressure in the pressure channel. The second pressure sensor is optionally configured to measure the pressure in the pressure channel. The first pressure sensor may be arranged in the reservoir housing accommodating the reservoir.

The irrigation system may comprise a user control interface housing with a user control interface. The user control interface enables a user to control the irrigation system. The user control interface housing may be attached to the tubing system. The first pressure sensor may be arranged in the user control interface housing. The user control interface housing may be arranged between the first end and the second end of the tubing system. In one or more exemplary irrigation systems, the pump may be arranged in the user control interface housing. The second pressure sensor may be arranged in the user control interface housing.

A second tubing part of the tubing system may be for connecting the user control interface housing with a catheter and may comprise at least a part of the pressure channel.

The tubing system may comprise a second membrane. The second membrane may be arranged in the pressure channel. The first pressure sensor may be configured to measure the pressure in the pressure channel via the second membrane. The second pressure sensor may be configured to measure the pressure in the pressure channel via the second membrane.

The pressure channel may be a closed or sealed channel. The pressure channel may be filled with gas, such as air. The pressure in the pressure channel may be in the range from 1.0 to 4.0 Psi, e.g. at 25°C.

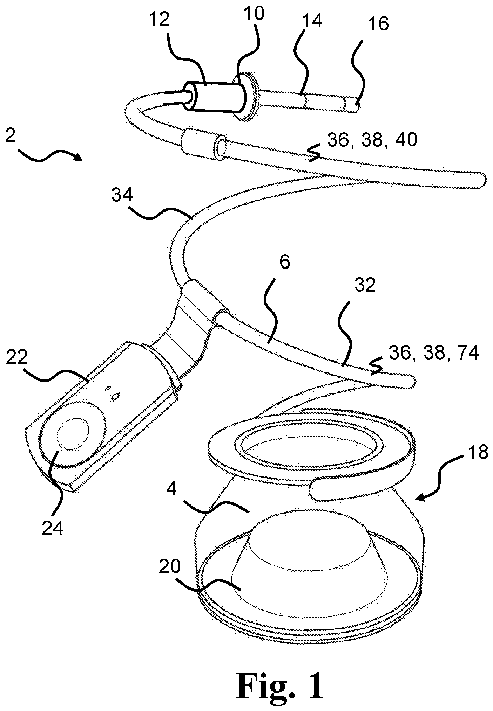

Fig. 1 shows an exemplary irrigation system for anal and/or stomal irrigation. The irrigation system 2 comprises a reservoir 4 for an irrigation liquid; a tubing system 6 with a first end 8 (not shown in Fig. 1) connectable to the reservoir 4, and a second end 10 with a second connector 12 and connectable to a catheter 14 for provision of a first fluid path for the irrigation liquid between the reservoir 4 and the catheter 14 comprising a distal end 16 for expelling irrigation liquid from the catheter 16. The irrigation system 2 comprises one or more pressure sensors including a first pressure sensor for provision of a first pressure signal. Further, the irrigation system 2 comprises a pump operable to pump the irrigation liquid from the reservoir 4 to the second end 10 of the tubing system 6. The pump is in irrigation system 2 arranged in reservoir housing 18 with reservoir 4, such as in bottom part 20 of the reservoir housing 18. The irrigation system 2 comprises a user interface housing 22 with a user interface 24 attached to the tubing system 6. The first pressure sensor is arranged in the user interface housing 22. The tubing system 6 comprises a first tubing part 32 and a second tubing part 34 each forming a part of the first fluid path of the irrigation system. The first tubing part 32 extends between the reservoir housing 18 and a user control interface housing 22 attached to the tubing system 6. The second tubing part 34 extends between the user control interface housing 22 and the second connector 12. The irrigation system 2 comprises a control system connected to the first pressure sensor and the pump. The control system is configured to operate the pump based on the first pressure signal from the first pressure sensor. The control system may be arranged in the user control interface housing 22 and/or in the reservoir housing 18.

The tubing system 6 comprises a pressure channel and a first membrane, wherein the first membrane is arranged in the second connector 12 of the tubing system between the first fluid path and the pressure channel. The pressure channel extends from the second connector 12 to the user interface housing 22 in the second tubing part 34, where the first pressure sensor is configured to measure the pressure in the pressure channel.

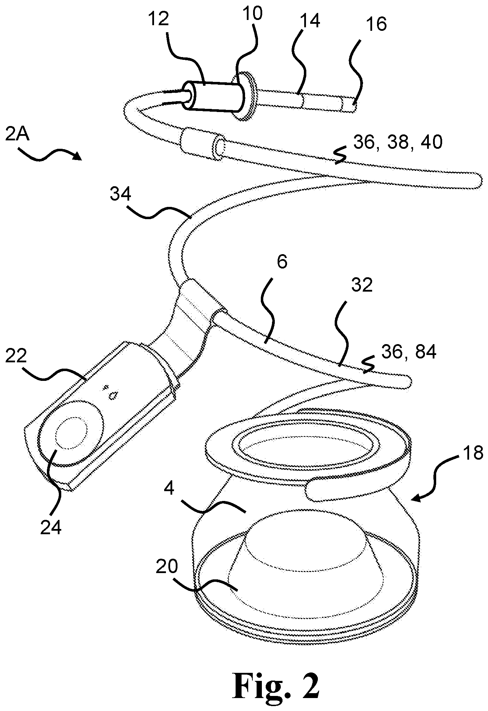

Fig. 2 shows an exemplary irrigation system for anal and/or stomal irrigation. The irrigation system 2A comprises a reservoir 4 for an irrigation liquid; a tubing system 6 with a first end 8 (not shown in Fig. 1) connectable to the reservoir 4, and a second end 10 with a second connector 12 and connectable to a catheter 14 for provision of a first fluid path for the irrigation liquid between the reservoir 4 and the catheter 14 comprising a distal end 16 for expelling irrigation liquid from the catheter 16. The irrigation system 2 comprises one or more pressure sensors including a first pressure sensor for provision of a first pressure signal. Further, the irrigation system 2 comprises a pump operable to pump the irrigation liquid from the reservoir 4 to the second end 10 of the tubing system 6. The irrigation system 2 comprises a user interface housing 22 with a user interface 24 attached to the tubing system 6. The pump and the first pressure sensor is in irrigation system 2A arranged in user interface housing 22. The tubing system 6 comprises a first tubing part 32 and a second tubing part 34 each forming a part of the first fluid path of the irrigation system. The first tubing part 32 extends between the reservoir housing 18 and a user control interface housing 22 attached to the tubing system 6. The second tubing part 34 extends between the user control interface housing 22 and the second connector 12. The irrigation system 2A comprises a control system connected to the first pressure sensor and the pump. The control system is configured to operate the pump based on the first pressure signal from the first pressure sensor. The control system may be arranged in the user control interface housing 22 and/or in the reservoir housing 18.

The tubing system 6 of irrigation system 2A comprises a pressure channel and a first membrane, wherein the first membrane is arranged in the second connector 12 of the tubing system between the first fluid path and the pressure channel. The pressure channel extends from the second connector 12 to the user interface housing 22 in the second tubing part 34, where the first pressure sensor is configured to measure the pressure in the pressure channel. The first tubing part 32 comprises a pump channel connected to the pump and the reservoir 4 for allowing the pump in the user control interface housing 22 to pump irrigation liquid from the reservoir 4 through the first fluid path to the catheter 14 connected to the second connector 12.

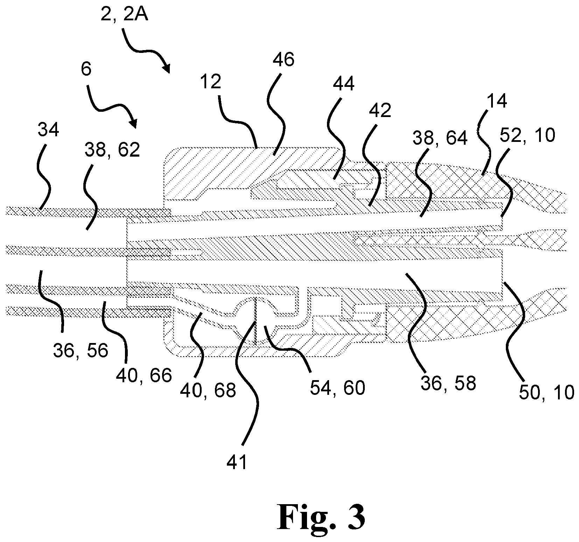

Fig. 3 illustrates a cross-sectional view of a part of an exemplary irrigation system. The irrigation system 2, 2A comprises a tubing system 6 with a first fluid path 36, a second fluid path 38, and a pressure channel 40. The first fluid path 36 is for feeding irrigation fluid from the reservoir to second end 10 of the tubing system, and the second fluid path 38 is for feeding second fluid to the second end 10 of the tubing system. The tubing system 6 comprises a second tubing part 34 and a second connector 12. The tubing system 6 comprises a first membrane 41 arranged between the first fluid path 36 and the pressure channel 40. The second connector comprises one or more connector parts including a first connector part 42, a second connector part 44 and a third connector part 46. The first connector part 42 is, in an assembled second connector 12, optionally snap-locked to and/or in threaded engagement with the second connector part 44. The second connector part 44 is, in an assembled second connector, optionally snap-locked to and/or in threaded engagement with the third connector part 46. The second connector 12 has a first outlet 50 of first fluid path 36 and a second outlet 52 of the second fluid path 38 (if present) of the tubing system 6. The first membrane 41 is arranged in an expansion chamber 54 of the first connector part 42. The first membrane 41 separates the first fluid channel 36 and the pressure channel 40. The first membrane is flexible to substantially equalize the pressure in the first fluid path and the pressure in the pressure channel, e.g. at least within a pressure difference less than a pressure difference threshold. The pressure difference threshold may be in the range from 0 to 0.5 psi. The pressure channel 40 may be a closed pressure channel, e.g. wherein the pressure in the closed pressure channel is in the range from 1.0 Psi to 4.0 Psi.

The second tubing part 34 comprises a first channel 56 and the first connector part 42 comprises a first channel 58, the channels 56, 58 forming a part of the first fluid path 36. The first channel 56 is in fluid communication with a first part 60 of the expansion chamber 54. The second tubing part 34 comprises a second channel 62 and the first connector part 42 comprises a second channel 64, the channels 62, 64 forming a part of the second fluid path 38. The second tubing part 34 comprises a pressure channel part 66 and the first connector part 42 comprises a pressure channel part 68 forming at least a part of the pressure channel 40.

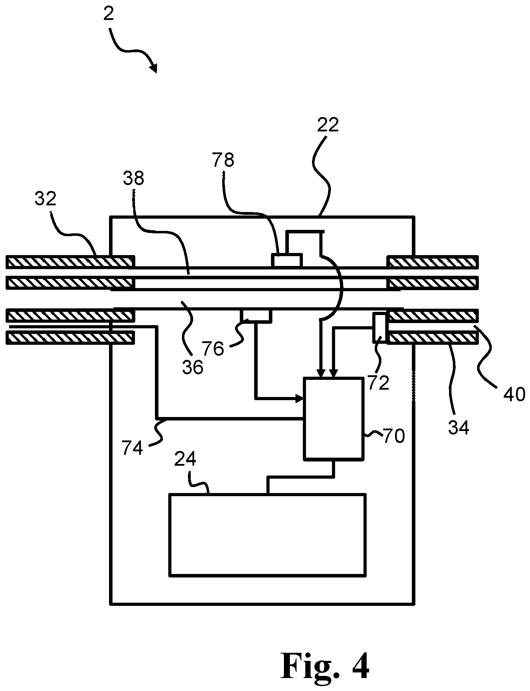

Fig. 4 schematically illustrates a part of an exemplary irrigation system. The irrigation system 2 comprises a tubing system including first tubing part 32 and second tubing part 34, the first and second tubing parts 32, 34 respectively connected to the user interface housing 22. A first fluid path 36 and a second fluid path 38 is provided in the irrigation system. The first fluid path 36 is used for feeding irrigation liquid from the reservoir to the catheter tip by connecting first outlet of the first fluid path to the catheter, see Fig. 3. The second fluid path 38 is used for feeding second fluid (for filling a catheter balloon) from the reservoir to the catheter balloon by connecting second outlet of the second fluid path to the catheter, see Fig. 3.

The irrigation system 2 comprises a control system including a controller 70, and a first pressure sensor 72 arranged in the user interface housing 22. The controller 70 is connected to the user interface 24 for receiving user input control signals and to the first pressure sensor 72 for receiving the first pressure signal indicative of the pressure in the pressure channel 40 from the first pressure sensor 72. Thus, the first pressure sensor 72 is configured to measure the pressure in the pressure channel 40. Arranging the first pressure sensor 72 in the user interface housing 22 is advantageous in order to provide a sufficiently accurate measurement of the pressure in the second connector 12. At the same time, the length of the pressure channel 40 is reduced, e.g. compared to a configuration with the first pressure sensor arranged in the reservoir housing. The controller 70 is connected to the pump arranged in the reservoir housing 18 by wired connection 74 and is configured to operate the pump (not shown) via one or more control signals on the wired connection 74. The wired connection 74 is optionally integrated in the first tubing part 32 as shown. The control system (controller 70) is configured to operate the pump (not shown) to reduce the irrigation liquid pressure in the first fluid path 36, if the pressure in the pressure channel 40 is larger than a first pressure threshold. The first pressure threshold may be 2.0 psi. In an exemplary irrigation system, the first pressure threshold is in the range from 1.6 to 2.2 psi.

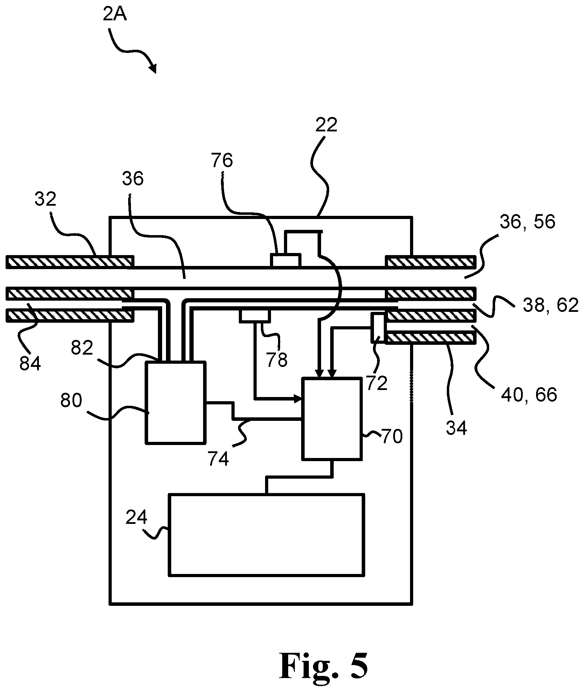

In Fig. 4 and 5, the first pressure sensor 72 seals the pressure channel 40 at a first end of the second tubing part 34 to form a closed pressure channel filled with gas.

The control system (controller 70) is configured to operate the pump based on the first pressure signal indicative of the pressure in the pressure channel 40. Thus, the pump is operated in accordance with the pressure in the first liquid path 36 close to the catheter tip 16. Thereby, the necessary safety margin can be reduced, and the irrigation system can operate closer to the maximum irrigation liquid pressure at the catheter tip in accordance with regulatory requirements. Thus, an improved and more safe irrigation is provided.

Optionally, the irrigation system 2 comprises a second pressure sensor 76 arranged in the user interface housing 22 and configured to measure the pressure in the first fluid path 36 in the user interface housing 22. The second pressure sensor 76 is connected to the controller 70 for provision of a second pressure signal indicative of the pressure in the first fluid path of the user interface housing 22. The control system (controller 70) is optionally configured to operate the pump based on the second pressure signal indicative of the pressure in the first fluid path of the user interface housing 22. Thus, an improved and more safe irrigation is provided and/or detection of errors/malfunction is improved.

Optionally, the irrigation system 2 comprises a third pressure sensor 78 arranged in the user interface housing 22 and configured to measure the pressure in the second fluid path 38 in the user interface housing 22. The third pressure sensor 78 is connected to the controller 70 for provision of a third pressure signal indicative of the pressure in the second fluid path of the user interface housing 22. The control system (controller 70) is optionally configured to operate the pump based on the third pressure signal indicative of the pressure in the second fluid path of the user interface housing 22. Thus, an improved and more safe filling of the catheter balloon is provided.

The controller 70 may be configured to receive one or more reservoir pressure signals from one or more pressure sensors arranged in the reservoir housing 18 via wired connection 74. The control system (controller 70) is optionally configured to operate the pump based on the reservoir pressure signal(s). Thus, an improved and more safe irrigation is provided and/or detection of errors/malfunction is improved.

Fig. 5 schematically illustrates a part of an exemplary irrigation system where the pump 80 is arranged in the user interface housing 22. The irrigation system 2A comprises a tubing system including first tubing part 32 and second tubing part 34, the first and second tubing parts 32, 34 respectively connected to the user interface housing 22. A first fluid path 36 and a second fluid path 38 is provided in the irrigation system. The first fluid path 36 is used for feeding irrigation liquid from the reservoir to the catheter tip by connecting first outlet of the first fluid path to the catheter, see Fig. 3. The second fluid path 38 is used for feeding second fluid (for filling a catheter balloon) from the user interface housing 22 to the catheter balloon by connecting second outlet of the second fluid path to the catheter, see Fig. 3.

A first outlet 82 of the pump 80 is connected to a pumping channel 84 of the first tubing part 32. The pumping channel 84 is connected or connectable to the reservoir of the reservoir housing. Thereby, the pump 80 is configured to apply a reservoir pressure in the reservoir, the reservoir pressure in the reservoir thereby pressing irrigation liquid through the first fluid path 36 towards the catheter 14, see Fig. 2.

A second outlet 86 of the pump 80 is connected to the second fluid path 38 (second channel 62 of the second tubing part 34. Thereby, the pump 80 is configured to fill the catheter balloon with air as second fluid via the second fluid path 38.

The irrigation system 2A comprises a control system including a controller 70, and a first pressure sensor 72 arranged in the user interface housing 22. The controller 70 is connected to the user interface 24 for receiving user input control signals and to the first pressure sensor 72 for receiving the first pressure signal indicative of the pressure in the pressure channel 40 from the first pressure sensor 72. Thus, the first pressure sensor 72 is configured to measure the pressure in the pressure channel 40. Arranging the first pressure sensor 72 in the user interface housing 22 is advantageous in order to provide a sufficiently accurate measurement of the pressure in the second connector 12. At the same time, the length of the pressure channel 40 is reduced, e.g. compared to a configuration with the first pressure sensor arranged in the reservoir housing. The controller 70 is connected to the pump 80 arranged in the user interface housing 22 by wired connection 74 and is configured to operate the pump 80 via one or more control signals on the wired connection 74. The control system (controller 70) is configured to operate the pump 80 to reduce the irrigation liquid pressure in the first fluid path 36, if the pressure in the pressure channel 40 is larger than a first pressure threshold. The first pressure threshold may be 2.0 psi.

The control system (controller 70) is configured to operate the pump based on the first pressure signal indicative of the pressure in the pressure channel 40. Thus, the pump 80 is operated in accordance with the pressure in the first liquid path 36 close to the catheter tip 16. Thereby, the necessary safety margin can be reduced, and the irrigation system can operate closer to the maximum irrigation liquid pressure at the catheter tip in accordance with regulatory requirements. Thus, an improved and more safe irrigation is provided.

Optionally, the irrigation system 2 comprises a second pressure sensor 76 arranged in the user interface housing 22 and configured to measure the pressure in the first fluid path 36 in the user interface housing 22. The second pressure sensor 76 is connected to the controller 70 for provision of a second pressure signal indicative of the pressure in the first fluid path of the user interface housing 22. The control system (controller 70) is optionally configured to operate the pump 80 based on the second pressure signal indicative of the pressure in the first fluid path of the user interface housing 22. Thus, an improved and more safe irrigation is provided and/or detection of errors/malfunction is improved.

Optionally, the irrigation system 2 comprises a third pressure sensor 78 arranged in the user interface housing 22 and configured to measure the pressure in the second fluid path 38 in the user interface housing 22. The third pressure sensor 78 is connected to the controller 70 for provision of a third pressure signal indicative of the pressure in the second fluid path of the user interface housing 22. The control system (controller 70) is optionally configured to operate the pump based on the third pressure signal indicative of the pressure in the second fluid path of the user interface housing 22. Thus, an improved and more safe filling of the catheter balloon is provided.

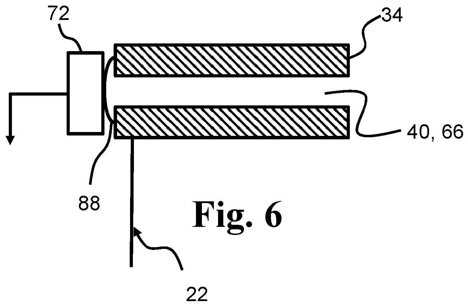

Fig. 6 shows a part of an exemplary irrigation system with a user interface housing 22, e.g. as shown in Fig. 4 or Fig. 5. The tubing system (second tubing part 34) is the same as in Fig. 4 or 5 with the additional feature, that the tubing system comprises a second membrane 88 arranged in the pressure channel 40. The second membrane 88 seals the pressure channel 40 at a first end of the second tubing part 34 to form a closed pressure channel filled with gas. The pressure in the pressure channel is in the range from 1.0 psi to 4.0 psi. The first pressure sensor 72 is configured to measure the pressure in the pressure channel 40 via the second membrane by the flexible second membrane 88 contacting and applying a pressure to the first pressure sensor 72.

Although particular features have been shown and described, it will be understood that they are not intended to limit the claimed invention, and it will be made obvious to those skilled in the art that various changes and modifications may be made without departing from the scope of the claimed invention. The specification and drawings are, accordingly to be regarded in an illustrative rather than restrictive sense.

## Claims

1. An irrigation system (2) for anal and/or stomal irrigation, the irrigation system (2) comprising:
- a reservoir (4) for an irrigation liquid;
- a tubing system (6) with a first end (8) connectable to the reservoir, and a second end (10) connectable to a catheter (14) for provision of a first fluid path (36) for the irrigation liquid between the reservoir (4) and the catheter (14), the catheter comprising a distal end (16) for expelling irrigation liquid from the catheter;
- one or more pressure sensors (72,76,78) including a first pressure sensor (72) for provision of a first pressure signal;
- a pump (80) operable to pump the irrigation liquid from the reservoir (4) to the second end (10) of the tubing system (6); and
- a control system connected to the first pressure sensor (72) and configured to operate the pump (80) based on the first pressure signal, **characterised in that** the tubing system (6) comprises a pressure channel (40) and a first membrane (41), wherein the first membrane (41) is arranged between the first fluid path (36) and the pressure channel (40), and the first pressure sensor (72) is configured to measure the pressure in the pressure channel.

2. Irrigation system according to claim 1, wherein the tubing system (6) comprises a second connector (12) at the second end (10), and the first membrane (41) is arranged in the second connector (12).

3. Irrigation system according to any of claims 1-2, wherein the irrigation system (2) comprises a user control interface housing (22) attached to the tubing system (2), wherein the first pressure sensor (72) is arranged in the user control interface housing (22).

4. Irrigation system according to any of claims 1-2, wherein the first pressure sensor (72) is arranged in a reservoir housing (18) accommodating the reservoir.

5. Irrigation system (2) according to any of claims 1-4, wherein the tubing system (6) comprises a second fluid path (38) parallel to the first fluid path (36) for feeding second fluid to the second end (10) of the tubing system (6).

6. Irrigation system according to any of claims 1-5, wherein the one or more pressure sensors (72,76,78) include a second pressure sensor (76) for provision of a second pressure signal, wherein the control system is connected to the second pressure sensor (76) and configured to operate the pump (80) based on the second pressure signal.

7. Irrigation system according to claim 6 as dependent on claim 3, wherein the second pressure sensor (76) is arranged in the user control interface housing (22).

8. Irrigation system according to any of claims 1-7, wherein the first pressure signal is indicative of relative pressure between the pressure in the pressure channel (40) and an ambient pressure.

9. Irrigation system according to any of claims 1-8, wherein the first pressure signal is indicative of absolute pressure in the pressure channel (40).

10. Irrigation system according to any of claims 1-9, wherein the tubing system (6) comprises a second membrane (88) arranged in the pressure channel (40), and wherein the first pressure sensor (72) is configured to measure the pressure in the pressure channel (40) via the second membrane (88).

11. Irrigation system according to any of claims 1-10, wherein the pressure channel (40) is filled with gas.

12. Irrigation system according to any of claims 1-11, wherein the first membrane (41) is arranged at a distance less than 50 cm from the second end (10) of the tubing system.

13. Irrigation system according to any of claims 1-12, wherein the control system is configured to operate the pump (80) to reduce the irrigation liquid pressure in the first fluid path (36), if the pressure in the pressure channel (40) is larger than a first pressure threshold.

## Patentansprüche

1. Spülsystem (2) zur Anal- und/oder Stomaspülung, wobei das Spülsystem (2) umfasst:
- ein Reservoir (4) für eine Spülflüssigkeit;
- ein Schlauchsystem (6) mit einem ersten Ende (8), das mit dem Reservoir verbindbar ist, und einem zweiten Ende (10), das mit einem Katheter (14) verbindbar ist, um einen ersten Fluidweg (36) für die Spülflüssigkeit zwischen dem Reservoir (4) und dem Katheter (14) bereitzustellen, wobei der Katheter ein distales Ende (16) zum Ausstoßen von Spülflüssigkeit aus dem Katheter umfasst;
- einen oder mehrere Drucksensoren (72, 76, 78), einschließlich eines ersten Drucksensors (72) zum Bereitstellen eines ersten Drucksignals;
- eine Pumpe (80), die funktionsfähig ist, die Spülflüssigkeit aus dem Reservoir (4) zu dem zweiten Ende (10) des Schlauchsystems (6) zu pumpen; und
- ein Steuersystem, das mit dem ersten Drucksensor (72) verbunden ist und dafür gestaltet ist, die Pumpe (80) auf der Grundlage des ersten Drucksignals zu betreiben, **dadurch gekennzeichnet, dass** das Schlauchsystem (6) einen Druckkanal (40) und eine erste Membran (41) umfasst, wobei die erste Membran (41) zwischen dem ersten Fluidweg (36) und dem Druckkanal (40) angeordnet ist und der erste Drucksensor (72) dafür gestaltet ist, den Druck in dem Druckkanal zu messen.

2. Spülsystem gemäß Anspruch 1, wobei das Schlauchsystem (6) ein zweites Verbindungselement (12) an dem zweiten Ende (10) umfasst und die erste Membran (41) in dem zweiten Verbindungselement (12) angeordnet ist.

3. Spülsystem gemäß einem der Ansprüche 1-2, wobei das Spülsystem (2) ein Benutzersteuerungs-Schnittstellengehäuse (22) umfasst, das an dem Schlauchsystem (2) angebracht ist, wobei der erste Drucksensor (72) in dem Benutzersteuerungs-Schnittstellengehäuse (22) angeordnet ist.

4. Spülsystem gemäß einem der Ansprüche 1-2, wobei der erste Drucksensor (72) in einem Reservoirgehäuse (18) angeordnet ist, das das Reservoir enthält.

5. Spülsystem (2) gemäß einem der Ansprüche 1-4, wobei das Schlauchsystem (6) einen zweiten Fluidweg (38) parallel zu dem ersten Fluidweg (36) umfasst, um dem zweiten Ende (10) des Schlauchsystems (6) ein zweites Fluid zuzuführen.

6. Spülsystem gemäß einem der Ansprüche 1-5, wobei der eine oder die mehreren Drucksensoren (72, 76, 78) einen zweiten Drucksensor (76) zum Bereitstellen eines zweiten Drucksignals umfassen, wobei das Steuersystem mit dem zweiten Drucksensor (76) verbunden ist und dafür gestaltet ist, die Pumpe (80) auf der Grundlage des zweiten Drucksignals zu betreiben.

7. Spülsystem gemäß Anspruch 6, wie abhängig von Anspruch 3, wobei der zweite Drucksensor (76) in dem Benutzersteuerungs-Schnittstellengehäuse (22) angeordnet ist.

8. Spülsystem gemäß einem der Ansprüche 1-7, wobei das erste Drucksignal für den relativen Druck zwischen dem Druck in dem Druckkanal (40) und einem Umgebungsdruck indikativ ist.

9. Spülsystem gemäß einem der Ansprüche 1-8, wobei das erste Drucksignal für den absoluten Druck in dem Druckkanal (40) indikativ ist.

10. Spülsystem gemäß einem der Ansprüche 1-9, wobei das Schlauchsystem (6) eine zweite Membran (88) umfasst, die in dem Druckkanal (40) angeordnet ist, und wobei der erste Drucksensor (72) dafür gestaltet ist, den Druck in dem Druckkanal (40) über die zweite Membran (88) zu messen.

11. Spülsystem gemäß einem der Ansprüche 1-10, wobei der Druckkanal (40) mit Gas gefüllt ist.

12. Spülsystem gemäß einem der Ansprüche 1-11, wobei die erste Membran (41) in einem Abstand von weniger als 50 cm von dem zweiten Ende (10) des Schlauchsystems angeordnet ist.

13. Spülsystem gemäß einem der Ansprüche 1-12, wobei das Steuersystem dafür gestaltet ist, die Pumpe (80) zu betreiben, um den Druck der Spülflüssigkeit in dem ersten Fluidweg (36) zu verringern, wenn der Druck in dem Druckkanal (40) höher als eine erste Druckschwelle ist.

## Revendications

1. Système d'irrigation (2) pour l'irrigation anale et/ou de stomie, le système d'irrigation (2) comprenant :
- un réservoir (4) pour un liquide d'irrigation ;
- un système de tubulure (6) avec une première extrémité (8) pouvant être reliée au réservoir, et une seconde extrémité (10) pouvant être reliée à un cathéter (14) pour fournir un premier trajet de fluide (36) pour le liquide d'irrigation entre le réservoir (4) et le cathéter (14), le cathéter comprenant une extrémité distale (16) pour expulser le liquide d'irrigation du cathéter ;
- un ou plusieurs capteurs de pression (72, 76, 78) comprenant un premier capteur de pression (72) pour fournir un premier signal de pression ;
- une pompe (80) pouvant fonctionner pour pomper le liquide d'irrigation du réservoir (4) vers la seconde extrémité (10) du système de tubulure (6) ; et
- un système de commande relié au premier capteur de pression (72) et configuré pour actionner la pompe (80) sur la base du premier signal de pression, **caractérisé en ce que** le système de tubulure (6) comprend un canal de pression (40) et une première membrane (41), la première membrane (41) étant agencée entre le premier trajet de fluide (36) et le canal de pression (40), et le premier capteur de pression (72) étant configuré pour mesurer la pression dans le canal de pression.

2. Système d'irrigation selon la revendication 1, le système de tubulure (6) comprenant un second connecteur (12) au niveau de la seconde extrémité (10), et la première membrane (41) étant agencée dans le second connecteur (12).

3. Système d'irrigation selon l'une quelconque des revendications 1 et 2, le système d'irrigation (2) comprenant un boîtier d'interface de commande utilisateur (22) fixé au système de tubulure (2), le premier capteur de pression (72) étant agencé dans le boîtier d'interface de commande utilisateur (22).

4. Système d'irrigation selon l'une quelconque des revendications 1 et 2, le premier capteur de pression (72) étant agencé dans un boîtier de réservoir (18) recevant le réservoir.

5. Système d'irrigation (2) selon l'une quelconque des revendications 1 à 4, le système de tubulure (6) comprenant un second trajet de fluide (38) parallèle au premier trajet de fluide (36) pour alimenter en second fluide la seconde extrémité (10) du système de tubulure (6).

6. Système d'irrigation selon l'une quelconque des revendications 1 à 5, le ou les capteurs de pression (72, 76, 78) comprenant un deuxième capteur de pression (76) pour fournir un deuxième signal de pression, le système de commande étant relié au deuxième capteur de pression (76) et configuré pour actionner la pompe (80) sur la base du deuxième signal de pression.

7. Système d'irrigation selon la revendication 6 lorsque dépendante de la revendication 3, le deuxième capteur de pression (76) étant agencé dans le boîtier d'interface de commande utilisateur (22).

8. Système d'irrigation selon l'une quelconque des revendications 1 à 7, le premier signal de pression étant indicatif de la pression relative entre la pression dans le canal de pression (40) et une pression ambiante.

9. Système d'irrigation selon l'une quelconque des revendications 1 à 8, le premier signal de pression étant indicatif de la pression absolue dans le canal de pression (40).

10. Système d'irrigation selon l'une quelconque des revendications 1 à 9, le système de tubulure (6) comprenant une seconde membrane (88) agencée dans le canal de pression (40), et le premier capteur de pression (72) étant configuré pour mesurer la pression dans le canal de pression (40) via la seconde membrane (88).

11. Système d'irrigation selon l'une quelconque des revendications 1 à 10, le canal de pression (40) étant rempli de gaz.

12. Système d'irrigation selon l'une quelconque des revendications 1 à 11, la première membrane (41) étant agencée à une distance inférieure à 50 cm de la seconde extrémité (10) du système de tubulure.

13. Système d'irrigation selon l'une quelconque des revendications 1 à 12, le système de commande étant configuré pour actionner la pompe (80) afin de réduire la pression du liquide d'irrigation dans le premier trajet de fluide (36), si la pression dans le canal de pression (40) est supérieure à un premier seuil de pression.
